(19) Europäisches Patentamt
European Patent Office
Office européen des brevets

(11) Veröffentlichungsnummer: **0 190 992**
**A2**

(12) # EUROPÄISCHE PATENTANMELDUNG

(21) Anmeldenummer: 86810041.3

(22) Anmeldetag: 24.01.86

(51) Int. Cl.[4]: C 07 D 303/22
C 07 C 143/14, C 11 D 1/18
C 11 D 1/92

(30) Priorität: 30.01.85 US 696552

(43) Veröffentlichungstag der Anmeldung:
13.08.86 Patentblatt 86/33

(84) Benannte Vertragsstaaten:
CH DE FR GB IT LI NL

(71) Anmelder: CIBA-GEIGY AG
Klybeckstrasse 141
CH-4002 Basel(CH)

(72) Erfinder: Falk, Robert A.
35 Glenside Drive
New City New York 10956(US)

(54) **Perfluoralkylalkylthio-,-sulfinyl- oder -sulfonylalkyl-glycidylether, Verfahren zu deren Herstellung und deren Verwendung.**

(57) Perfluoralky-alkyl-thio-, -sulfinyl- oder -sulfonylalkyl-glycidyl-ether der Formel I

$$R_f-R_1-S(O)_m-R-OCH_2CH\overset{O}{\text{——}}CH_2 \quad (I),$$

worin

$R_f$ Perfluoralkyl oder Perfluoralkoxyperfluoralkyl mit 3 bis 18 C-Atomen ist,

$R_1$ Alkylen, Carbamidoalkylen oder Sulfonamidoalkylen mit bis zu 6 C-Atomen darstellt, worin das N-Atom der Amidogruppe durch Wasserstoff oder Niederalkyl substituiert ist, m 0, 1 oder 2 bedeutet, und

R Akylen mit 2 bis 12 C-Atomen ist, sind wertvolle Zwischenprodukte für die Herstellung von oberflächenaktiven Stoffen.

EP 0 190 992 A2

Croydon Printing Company Ltd.

CIBA-GEIGY AG
Basel (Schweiz)

6-15241/=/CGC 1120

Perfluoralkylalkylthio-, -sulfinyl- oder -sulfonylalkyl-glycidylether, Verfahren zu deren Herstellung und deren Verwendung

---

Die vorliegende Erfindung betrifft Perfluoralkyl-alkylthio-, -sulfinyl- oder -sulfonyl-alkyl-glycidylether der Formel I

$$R_f-R_1-S(O)_m-R-OCH_2CH\underset{O}{\frown}CH_2 \qquad (I),$$

worin

$R_f$ lineares oder verzweigtes Perfluoralkyl oder Perfluoralkoxyperfluoralkyl mit 3 bis 18 C-Atomen ist,

$R_1$ lineares oder verzweigtes Alkylen, Carbamidoalkylen oder Sulfonamidoalkylen mit bis zu 6 C-Atomen darstellt, wobei das N-Atom der Amidogruppe durch Wasserstoff oder Niederalkyl substituiert ist,

m für 0,1 oder 2 steht, und

R lineares oder verzweigtes $C_2$-$C_{12}$-Alkylen ist.

Niederalkyl bedeutet Alkylgruppen, die 1 bis 6, bevorzugt 1 bis 4 C-Atome enthalten.

$R_f$ ist bevorzugt Perfluoralkyl, besonders lineares Perfluoralkyl.

Der Rest $R_f$ enthält bevorzugt 3 bis 16, insbesondere 6 bis 12 C-Atome. Mischungen von Verbindungen der Formjel I in denen $R_f$ einen unterschiedlichen Gehalt an C-Atomen aufweist, sind ebenfalls vorteilhaft.

$R_1$ ist bevorzugt lineares Alkylen, das 2 bis 6, besonders 2 bis 4 C-Atome enthalten kann. Besonders bevorzugt ist $R_1$ Ethylen.

In Formel I bedeutet m bevorzugt 0 oder 2.

R stellt bevorzugt lineares oder verzweigtes $C_2$-$C_6$-, besonders $C_2$-$C_4$-Alkylen dar. Besonders bevorzugt ist R lineares oder verzweigtes $C_3$-Alkylen.

Die Verbindungen der Formel I können nach an sich bekannten Verfahren hergestellt werden.

Die Verbindungen der Formel I mit m = 0 können z.B. hergestellt werden, indem man ein Merkaptan der Formel II

$$R_f\text{-}R_1\text{-}SH \qquad (II),$$

worin $R_f$ und $R_1$ wie zuvor definiert sind, mit einem Alkenylglycidylether der Formel III

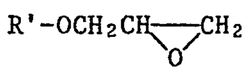

(III),

worin R' $C_2$-$C_{12}$-Alkenyl ist, umsetzt. Die Umsetzung wird vorteilhaft in Gegenwart eines freie Radikale erzeugenden Karalysators durchgeführt. Beispiele für solche Katalysatoren sind Azoverbindungen.

Die Wahl der Reaktionstemperatur und der Azoverbindung als Katalysator sind voneinander abhängig. Im Temperaturbereich von 40 bis 100°C werden wenig ungewünschte Nebenprodukte gebildet und die Reaktionsprodukte sind stabil. Um bei dieser Temperatur eine ausreichende Reaktionsgeschwindigkeit zu erzielen, ist es vorteilhaft, eine Azoverbindung als Katalysator zu verwenden, die in diesem Temperaturbereich im ausreichenden Mass zerfällt. Es werden daher Azoverbindungen bevorzugt, deren Halbwertszeit im Bereich von 40 bis 100°C eine Stunde beträgt.

Die Reaktion kann in Gegenwart eines inerten Lösungsmittels durchgeführt werden. Geeignete Lösungsmittel sind zum Beispiel aliphatische und aromatische Kohlenwasserstoffe wie Heptan, Methylcyclohexan, Benzol oder Toluol; chlorierte oder fluorierte aliphatische oder aromatische Kohlenwasserstoffe wie Methylenchlorid, Chloroform, Tetrachlor-Kohlenstoff, Trichlorethylen, Perchlorethylen, Freone (1,1,2-Trifluor-1,1,2-trichlorethan), Chlorbenzol, Trifluorbenzol, Hexafluorxylol; Ketone, Ester und Ether wie Aceton, Methylisobutylketon, Ethylacetat und höhere Homologe, Dialkylether, Tetrahydrofuran, Ethylenglycolmonomethyl- oder -monoethylether, Ethylenglycoldimethyl- oder -diethylether; Nitrile wie Acetonitril.

Wenn möglich wird die Reaktion vorzugsweise ohne Verwendung eines Lösungsmittels durchgeführt. Eine als Katalysator geeignete Azoverbindung ist 2,2'-Azo-bis(2,4-dimethylvaleronitril).

Ein anderes Herstellungsverfahren für Verbindungen der Formel I besteht darin, dass man einen Alkohol der Formel IV

$R_f$-$R_1$-S(O)$_m$-R-OH (IV),

worin $R_f$, $R_1$, R und m wie zuvor definiert sind, mit einem Epihalohydrin der Formel V

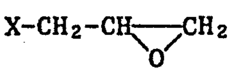 (V),

worin X Halogen, bevorzugt Chlor bedeutet, zu einem Halohydrin der Formel VI

$R_f$-$R_1$-S(O)$_m$-R-OCH$_2$CH-CH$_2$X (VI),
OH

worin $R_f$, $R_1$, R, m und wie zuvor definiert sind, umsetzt, und danach die Verbindung der Formel VI zur Bildung des gewünschten Glycidylethers der Formel I dehydrohalogeniert.

Diese Reaktion kann in Gegenwart eines inerten Lösungsmittels durchgeführt werden. Geeignete Lösungsmittel sind aprotische Lösungsmittel, zum Beispiel Ketone wie Aceton oder Methylethylketon; Ether wie Diethylether, Ethylenglycoldimethylether oder Tetrahydrofuran; Ester wie Ethylacetat oder Methylcellosolveacetat; aromatische Kohlenwasserstoffe wie Toluol; und Amide bzw. Lactame wie Dimethylformamid und N-Methylpyrrolidon. Vorteilhaft wird eine Lewis-Säure als Katalysator mitverwendet, zum Beispiel Bortrifluorid (als Diethyletherkomplex) oder Aluminiumtrichlorid, um die Bildung des Halohydrins der Formel VI zu beschleunigen. Falls die Reaktion ohne Lösungsmittel durchgeführt wird, liegt die Reaktionstemperatur vorteilhaft über dem Schmelzpunkt des Bortrifluorid-Etherates. Die Reaktion ist normalerweise exotherm. Im allgemeinen müssen die Halohydrine der Formel IV nicht isoliert werden und die Dehydrohalogenierung kann nach Beendigung der Reaktion im gleich Reaktionsgefäss vorgenommen werden.

Die Dehydrohalogenierung des Halohydrins der Formel VI wird vorteilhaft dergestalt durchgeführt, dass man auf das Halohydrin der Formel VI einer Base einwirken lässt, wobei bevorzugt eine stöchiometrische Menge oder ein geringer Ueberschuss der Base verwendet wird.

Geeignete Basen sind z.B. Natrium- und Kaliumhydroxid, Pyridin, Lutidin und Triethylamin. Vorteilhaft wird ein Lösungsmittel mitverwendet, das die Reaktanden und Endprodukte löst, aber nicht die gebildeten Salze, so dass das Salz ausfällt und nach üblichen Methoden, wie zum Beispiel Filtration, abgetrennt werden kann.

Die Dehydrohalogenierung wird im allgemeinen bei einer Temperatur von etwa 20 bis 100°C in einem wässrig-organischen Medium, zum Beispiel Wasser/Niederalkanol, oder wässrigen Kohlenwasserstoff-Medium, z.B. Wasser/Toluol, durchgeführt. Wenn die Reaktion in einem Zweiphasensystem durchgeführt wird, verwendet man vorteilhaft einen

Phasentransferkatalysator wie z.B. Didodecyldimethylammoniumhydroxid oder Tetrabutylammoniumhydrogensulfat, um die Reaktion zu beschleunigen.

In einer Ausführungsform des Verfahrens wird die Base, zum Beispiel eine 50%-ige wässrig NaOH-Lösung, langsam zum Reaktionsgemisch gegeben. Die Reaktion ist exotherm und die Reaktionstemperatur wird unter Rühren der Reaktionsmischung zwischen etwa 20 bis 60°C gehalten, bis das gewünschte Epoxid der Formel I gebildet ist. Die Reaktionszeit beträgt im allgemeinen 1 bis 3 Stunden.

Das ausgefallene Salz wird dann abfiltriert und vom Filtrat das Lösungsmittel durch Destillation im Vakuum entfernt. Der Destillationsrückstand enthält das gewünschte Epoxid der Formel I. Der Rückstand kann ohne weitere Reinigung verwendet, oder falls gewünscht, destillativ gereinigt werden.

Die erfindungsgemässen Epoxide der Formel I sind sehr reaktiv und können zur Herstellung von oberflächenaktiven, Perfluoralkylgruppen enthaltenden Stoffen (Tenside) verwendet werden.

Die Verbindungen der Formel I können hierzu mit aminischen Schwefeltrioxid-Komplexen umgesetzt werden, z.B. Trimethylaminx$SO_3$, N-Methylpyrolidonx$SO_3$, wobei man Tenside erhält, die eine niedrige Oberflächenspannung und Grenzflächenspannung aufweisen. Diese Reaktion ist im U.S. Patent 4,435,330 beschrieben. Die so erhaltenen Sulfato-Betain-Tenside können in Reinigungsmitteln, als Stabilisiermittel in Bodenwachsen oder als Netzmittel in wässrigen Feuerlöschmitteln verwendet werden.

Geeignete aminische Schwefeltrioxidkomplexe sind solche der Formel VII

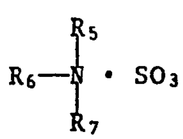

(VII),

worin $R_5$, $R_6$ und $R_7$ unabhängig Niederalkyl, und $R_6$ auch Benzyl darstellen, oder $R_6$ und $R_7$ zusammen Pentamethylen oder 3-Oxapentylen oder $R_5$, $R_6$ und $R_7$ zusammen mit dem N-Atom Pyridyl, Acridyl oder Chinolyl sind. Die Reaktion wird vorteilhaft unter Verwendung stöchiometrischer Mengen bei Temperaturen von 30 bis 180°C durchgeführt, gegebenenfalls in Gegenwart eines Lösungsmittels wie z.B. N-Methylpyrrolidon.

In einer anderen Ausführungsform können die Verbindungen der Formel I mit einer Aminosäure der Formel VIII

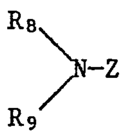

(VIII),

worin $R_8$ und $R_9$ Wasserstoff oder Niederalkyl sind und Z $C_1$-$C_{12}$-Alkylen bedeutet, das durch Carboxy, Sulfo, Phosphoro oder Phosphono substituiert ist, umsetzt. Geeignete Verbindungen der Formel VIII sind z.B. Glycin, Alanin, Asparaginsäure, Sarcosin, 2-Aminoethylhydrogenphosphat, Taurin oder Methyltaurin.

Sulfo, Phosphoro und Phosphono bedeuten saure Gruppen, die über ihre S- oder P-Atome oder über O-Atome an das Alkylen gebunden sind. Beispiele sind $-S(O)OH$, $-S(O)_2OH$, $-OS(O)OH$, $-OS(O)_2OH$, $-P(OH)_2$, $-P(O)(OH)_2$, $-OP(OH)_2$ und $-OP(O)(OH)_2$.

Die Addition der Epoxide der Formel I an die Aminosäuren der Formel VIII ist eine basenkatalysierte Reaktion in bevorzugt einem Einphasen-Reaktionsmedium. Dieses wird z.B. durch Wasser/Lösungsmittel-Mischungen erhalten, wobei man mit Wasser mischbare Lösungsmittel wie Methanol, Isopropanol oder Butoxyethanol verwendet. Die Reaktionstemperatur kann 25 bis 200°C, bevorzugt 50 bis 130°C betragen.

Die Zugabe eines basischen Katalysators erübrigt sich, wenn ein Reaktant genügend basisch ist. Alkalische Reaktionsbedingungen erzielt man durch Zugabe von Alkalihydroxiden, Ionenaustauschern oder nichtreaktiven Basen wie Triisopropylamin.

Die Produkte können direkt verwendet oder zuvor isoliert werden. Die Isolierung erfolgt je nach pH-Wert als Säuren, als isoelektronische neutrale Salze, oder als Alkalimetall- oder Ammoniumsalze. Die N-quaternären Derivate, die N-Niederalkyl oder N-Benzyl funktionell sind, werden als neutrale zwitterionische Verbindungen oder Säureaddukte, z.B. den Hydrochloriden, isoliert.

Die erhaltenen amphoteren Verbindungen sind hydrolysestabil und finden zahlreiche Verwendungsmöglichkeiten als Tenside und Netzmittel.

Die Alkohole der Formel IV sind bekannt oder können nach an sich bekannten Verfahren hergestellt werden, z.B. durch die Reaktion eines Merkaptans der Formel II mit einem Haloalkohol der Formel IX

X-R-OH (IX)

worin X und R wie zuvor definiert sind, oder mit einem ungesättigten Alkohol der Formel X

R'-OH (X),

worin R' wie zuvor definiert ist.

Die Verbindungen II und IX können in stöchiometrischen Mengen mit oder ohne Lösungsmittel in Gegenwart einer Base zur Bindung der gebildeten Halogenwasserstoff HX bei Temperaturen von 30 bis 120°C und Entfernen des gebildeten Salzes durch z.B. Waschen mit Wasser miteinander umgesetzt werden. Geeignete Basen sind Alkalimetallhydroxide und -carbonate, Erdalkalimetallhydroxide, Carbamate und Amine wie z.B. Trimethylamin und Pyridin. Vorteilhaft werden solche

Mengen Base zugegeben, dass der gesamte Halogenwasserstoff HX gebunden werden kann. Geeignete inerte Lösungsmittel sind z.B. Tetrahydrofuran, Dimethylsulfoxid und Niederalkanole.

Die Reaktion des Merkaptans der Formel II mit dem ungesättigten Alkohol der Formel X wird zweckmässig in Gegenwart eines Radikalinitiators, z.B. einer Azoverbindung wie 2,2'-Azobis-(2,4-dimethylvaleronitril), bei einer Temperatur von 30 bis 100°C durchgeführt. Die Reaktion kann mit oder ohne inerte Lösungsmittel durchgeführt werden. Geeignete Lösungsmittel sind beispielsweise Tetrahydrofuran, Methylethylketon oder Dimethylsulfoxid.

Zur Herstellung von Verbindungen der Formel IV, worin m 1 oder 2 ist, aus den entsprechenden Thioetheralkoholen, worin m 0 ist, kann der Thioetheralkohol mit einem Oxidationsmittel oxidiert werden. Geeignete Oxidationsmittel sind z.B. Wasserstoffperoxid in einem organischen sauren Medium wie Essigsäure. Die Reaktion wird bei einer Temperatur von 30 bis 100°C durchgeführt, bis das entsprechende Sulfoxid oder Sulfon gebildet ist. Im allgemeinen wird bei niedrigeren Temperaturen, z.B. 30 bis 50°C, die Bildung des Sulfoxides begünstigt, während bei erhöhten Temperaturen, z.B. 50 bis 100°C, die Bildung des Sulfons begünstigt wird. Die Bildung des Sulfoxides kann auch durch die Beschränkung der Peroxidmenge auf ein molares Verhältnis von 1:1 begünstigt werden, während ein wesentlicher Ueberschuss an Peroxid die Bildung des Sulfon begünstigt.

Aehnlich können auch die Verbindungen der Formel I, worin m 0 ist, in die entsprechenden Sulfoxide und Sulfone überführt werden, indem man die Thioether mit geeigneten Oxidationsmitteln, die nicht die Glycidylgruppe beeinflussen, behandelt. Solche Oxidationsmittel sind beispielsweise Perbenzoesäuren wie Metachlorperbenzoesäure. Die Reaktion wird zweckmässig in einem inerten Lösungsmittel, z.B. halogenierten Kohlenwasserstoffen wie Chloroform, bei Temperaturen von 10 bis 50°C durchgeführt. Auch hier begünstigen erhöhte Temperaturen und ein Ueberschuss an Peroxid die Bildung von Sulfonen.

Die nachfolgenden Beispiele erläutern die Erfindung näher. Teile sind hierin Gewichtsteile, wenn nicht anders erwähnt.

Beispiel 1: Eine Mischung von 1-Allyloxy-2,3-epoxypropan (24,1 g; 0,212 mol) und 2,2'-Azobis(2,4-dimethylvaleronitril) (0,99 g) wird bei 60 bis 75°C unter Stickstoff innerhalb von 40 Minuten tropfenweise zu einer Lösung von 1,1,2,2-Tetrahydroperfluoroktanthiol (77,26 g; 0,202 Mol) in 11 g Toluol gegeben. Die Reaktionsmischung wird 3 Stunden bei 65 bis 70°C gerührt und danach das Toluol bei 80°C unter Vakuum (33 mbar) abdestilliert. Man erhält 99,2 g (99 % der Theorie einer klaren, farblosen Flüssigkeit, die im Verhältnis 10:1 die Isomeren I und II enthält:

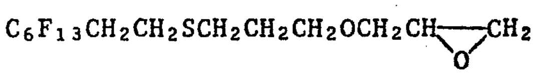 (I)

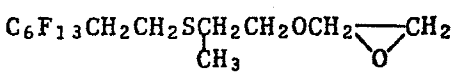 (II) .

Die Titration mit Perchlorsäure in Gegenwart von Tetrabutylammoniumjodid ergibt ein Aequivalentgewicht von 509 (Theorie: 494). Das $^1H$-NMR-Spektrum zeigt Protonen Resonanzen bei δ 1,85, 2 Protonen ($-SCH_2\underline{CH_2}CH_2O$); δ 2,37, 2 Protonen ($C_6F_{13}\underline{CH_2}CH_2$); δ 2,69, 6 Protonen ($C_6F_{13}CH_2\underline{CH_2}S\underline{CH_2}$ 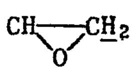); δ 3,11, 1 Proton

(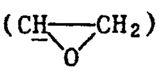); δ 3,33 and δ 3,71, 2 Protonen ($O-\underline{CH_2}$); and δ 3,57,

2 Protonen($\underline{CH_2}OCH_2$).

Elementaranalyse ($C_{14}H_{15}F_{13}O_2S$):
Berechnet: C: 34,0; H: 3,0
Gefunden: C: 33,9; H: 3,0.

Example 2: 1,1,2,2-Tetrahydroperfluoralkylthiol (Verteilung für Perfluoralkyl: 2,1 % $C_4$, 37,6 % $C_6$, 34,3 % $C_8$, 19,0 % $C_{10}$, 5,5 % $C_{12}$, 1,0 % $C_{14}$) (279,6 g; 0,615 mol) und eine Lösung von 1-Allyloxy-2,3-epoxypropan (73,6 g; 0,696 mol) und 2,2'-Azobis-(2,4-dimethylvaleronitril) (3,05 g) werden bei 60°C unter Stickstoff innerhalb 90 Minuten durch getrennte Tropftrichter in ein Reaktionsgefäss getropft. Danach wird die Reaktionsmischung eine Stunde lang bei

60°C gerührt. Man erhält 342,7 g (98 % der Theorie) eines leicht gelben Gels. Eine Gelchromatographie-Analyse ergibt, dass 99 % davon die Isomeren I und II im Verhältnis 10:1 sind:

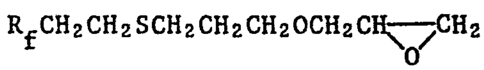 (I)

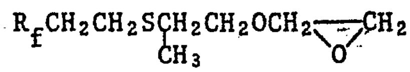 (II).

Die Titration mit Perchlorsäure in Gegenwart von Tetrabutylammoniumjodid ergibt ein Aequivalentgewicht (mol) von 573 (Theorie: 569). Das $^1$H-NMR-Spektrum zeigt die gleichen Protonenresonanzen wie in Beispiel 1.

Beispiel 3: 1,1,2,2-Tetrahydroperfluoralkylthiol (Verteilung für Perfluoralkyl: 0,2 % $C_4$, 0,4 % $C_6$, 8,4 % $C_8$, 28,0 % $C_{10}$, 39,1 % $C_{12}$, 18,1 % $C_{14}$, 4,3 % $C_{16}$) (90,0 g. 0,138 mol) wird parallel mit einer Lösung von 2,2-Azobis-(2,4-dimethylvaleronitril) (0,8 g) in 16,5 g (0,145 mol) 1-Allyloxy-2,3-epoxypropan in zwei Behälter eingewogen und bei 65-70°C unter Stickstoff in ein Reaktionsgefäss gegeben, in dem 24 g Toluol vorgelegt sind. Die Reaktionsmischung wird 90 Minuten bei 85°C gerührt und danach das Toluol bei Raumtemperatur im Vakuum abgezogen. Man erhält 100,4 g (95 % der Theorie) eines weissen kristallinen Festkörpers mit einem Schmelzbereich von 78 bis 107°C. Eine Gelchromatographie-Analyse ergibt 99 % Gehalt an den Isomeren I und II im Verhältnis 10:1:

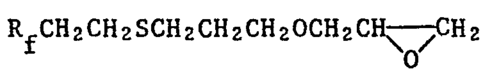 (I)

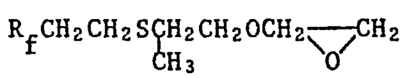 (II).

Die Titration mit Perchlorsäure in Gegenwart von Tetrabutylammoniumjodid ergibt ein Aequivalentgewicht (mol) von 756 (Theorie: 767).

Beispiel 4: Eine Lösung von 64,8 g (0,3 mol) m-Chlorperbenzoesäure in 800 ml Chloroform wird unter Stickstoff tropfenweise während 5 Stunden zu einer Isomeren-Mischung von 1-[(1,1,2,2-Tetrahydroperfluoroctylthio)-1-propyloxy]-2,3-epoxypropan und 1-[(1,1,2,2-Tetrahydroperfluoroctylthio]-2-propyloxy]-2,3-epoxypropan (74,1 g,

0,15 mol), die in 400 ml Chloroform gelöst ist, gegeben. Die Reaktionsmischung wird während der Zugabe bei 25 bis 30°C gehalten und über Nacht bei Raumtemperatur gerührt. Die ausgefallene m-Chlorbenzoesäure wird abfiltriert. Das Filtrat wird zweimal mit eiskalter 10%-iger wässriger NaOH, einmal mit eiskalter Salzlauge und einmal mit Eiswasser gewaschen. Die organische Phase wird über $MgSO_4$ getrocknet, das Lösungsmittel verdampft und über Nacht im Hochvakuum getrocknet. Man erhält 73,5 g (93 % der Theorie) eines weissen kristallinen Festkörpers mit einem Schmelzpunkt von 61-64°C. Die Gelchromatographie-Analyse ergibt 94 % Gehalt der Isomeren I und II im Verhältnis 10:1:

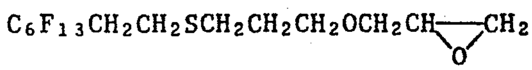 (I)

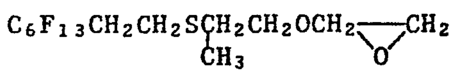 (II).

Die Titration mit Perchlorsäure in Gegenwart von Tetrabutylammoniumjodid ergibt ein Aequivalentgewicht (mol) von 534 (Theorie: 526). Das $^1$H-NMR-Spektrum zeigt Protonen Resonanzen bei δ 2,12, 2 Protonen ($SO_2CH_2C\underline{H}_2CH_2O$); δ 2,57, 1 proton (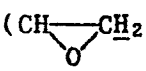);
δ 2,76, 2 Protonen ($C_6F_{13}C\underline{H}_2$); δ 2,77, 1 Proton (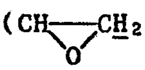);
δ 3,11, 1 Proton (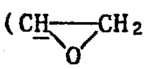); δ 3,23 4-Protonen ($C_6F_{13}CH_2C\underline{H}_2SO_2C\underline{H}_2$);
δ 3,31, 1 Proton ($O-C\underline{H}_2CH$); δ 3,63 2 Protonen ($SO_2CH_2CH_2C\underline{H}_2$) and δ 3,79, 1 Proton ($O-C\underline{H}_2CH$).

Elementaranalyse für $C_{14}H_{15}F_{13}O_4S$

Berechnet: C 31,9; H 2,85

Gefunden: C 33,1; H 2,9.

<u>Beispiel 5:</u> Eine isomere Mischung (10:1) von 1-[(1,1,2,2-Tetrahydroperfluoroctylthio)-1-propyloxy]-2,3-epoxypropan und 1-[(1,1,2,2-Tetrahydroperfluoroctylthio)-2-propyloxy]-2,3-epoxypropan (14,8 g 0,03 mol) in 42,2 g 2-Propanol gelöst und 10 g deionisiertes Wasser werden bei 60°C 5 Stunden unter Stickstoff mit dem Natriumsalz von N-Methyltaurin (11,4 g 42,2 % in $H_2O$) umgesetzt. Die klare gelbe

Lösung wird dialysiert (24 Stunden), das Wasser bei 80°C in einem Ofen entfernt, der gelbe Rückstand in heissem Aceton gelöst, das Aceton verdampft und der Rückstand unter Hochvakuum über Nacht getrocknet. Man erhält einen amorphen Rückstand.

Elementaranalyse für $C_{17}H_{23}F_{13}NOP_5S_2Na$:

| | | | | |
|---|---|---|---|---|
| Berechnet: | C 31,8; | H 3,58; | N 2,18; | F 38,5 |
| Gefunden: | C 31,6; | H 3,5; | N 2,0; | F 38,5 |

Der Rückstand besteht aus einer Isomerenmischung von

$C_6F_{13}CH_2CH_2SCH_2CH_2CH_2OCH_2\underset{OH}{\underset{|}{C}}HCH_2\underset{CH_3}{\underset{|}{N}}CH_2CH_2SO_3Na$ und

$C_6F_{13}CH_2CH_2S\underset{CH_3}{\underset{|}{C}}HCH_2OCH_2\underset{OH}{\underset{|}{C}}HCH_2\underset{CH_3}{\underset{|}{N}}CH_2CH_2SO_3Na$ .

Beispiel 6:

Tensideigenschaften des Gemisches gemäss Beispiel 5

| Schaumhöhe bei 0.1 %[1] in | | | Benetzung (Draves) | Oberflächenspannung[3] (mN/m) | | | Grenzflächenspannung[4] (mN/m) | | |
|---|---|---|---|---|---|---|---|---|---|
| Deionisiertem Wasser | 300 ppm NaCl | Sea water | in Sek.[2] | 0,1% | 0,01 % | 0,001 % | 0,1 % | 0,01 % | 0,001 % |
| 178/155 | 163/153 | 78/64 | 218 | 185 | 180 | 293 | 60 | 78 | 164 |

1) ASTM-Method D-1173-53, bei Raumtemperatur, Schaumhöhe in mm zu Begin und nach 5 Minuten
2) ASTM-Method D-2281-68, Sinkzeit in Sekunden
3) ASTM-Method D-1331-56, DuNouy Tensiometer
4) ASTM-Method D-1331-56, DuNouy Tensiometer

Beispiele 7-10: Gemäss den Verfahren der Beispiele 1-6 werden die folgenden Glycidylether und oberflächenaktiven Substanzen hergestellt.

| Example | Glycidylether | Oberflächenaktive Substanz |
|---|---|---|
| 7 | 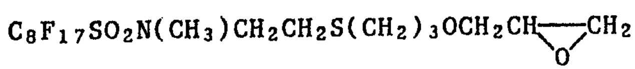 | 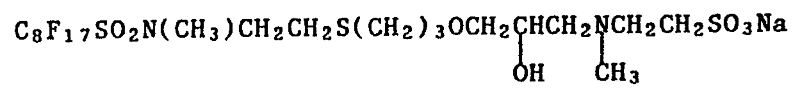 |
| 8 | 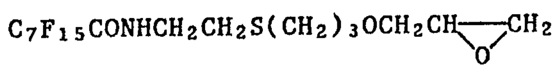 | 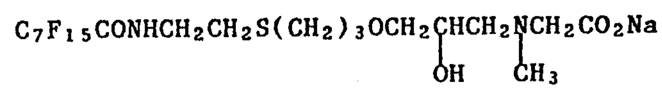 |
| 9 | 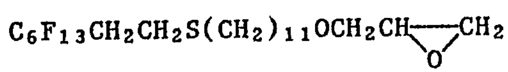 | 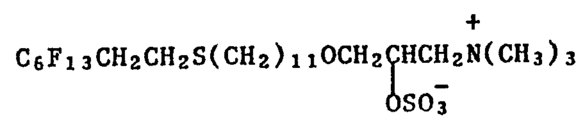 |
| 10 | 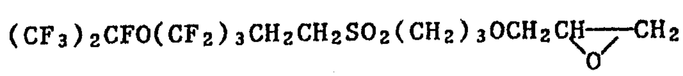 | 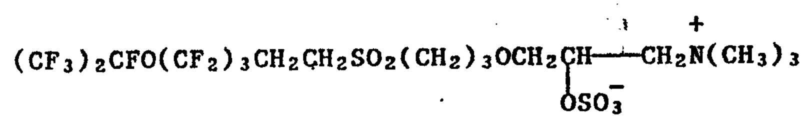 |

Patentansprüche

1. Eine Verbindung der Formel I

$$R_f\text{-}R_1\text{-}S(O)_m\text{-}R\text{-}OCH_2\underset{\diagdown O \diagup}{CH\text{—}CH_2} \qquad (I),$$

worin

$R_f$ lineares oder verzweigtes Perfluoralkyl oder Perfluoralkoxyperfluoralkyl mit 3 bis 18 C-Atomen ist,

$R_1$ lineares oder verzweigtes Alkylen, Carbamidoalkylen oder Sulfonamidoalkylen mit bis zu 6 C-Atomen darstellt, wobei das N-Atom der Amidogruppe durch Wasserstoff oder Niederalkyl substituiert ist,

m für 0,1 oder 2 steht, und

R lineares oder verzweigtes $C_2$-$C_{12}$-Alkylen ist.

2. Eine Verbindung gemäss Anspruch 1, worin $R_f$ in Formel I für Perfluoralkyl steht.

3. Eine Verbindung gemäss Anspruch 2, worin in Formel I $R_f$ für lineares Perfluoralkyl mit 6 bis 12 C-Atomen steht.

4. Eine Verbindung gemäss Anspruch 1, worin $R_1$ in Formel I lineares Alkylen mit 2 bis 6 C-Atomen ist.

5. Eine Verbindung gemäss Anspruch 4, worin in Formel I $R_1$ für Ethylen steht.

6. Eine Verbindung gemäss Anspruch 1, worin m in Formel I 0 oder 2 ist.

7. Eine Verbindung gemäss Anspruch 1, worin R in Formel I lineares oder verzweigtes $C_3$-Alkylen ist.

8. Eine Verbindung gemäss Anspruch 7, worin $R_1$ in Formel I Ethylen und m 0 ist.

9. Verfahren zur Herstellung von Verbindungen der Formel I gemäss Anspruch 1,

$$R_f-R_1-S(O)_m-R-OCH_2\underset{O}{CH-CH_2} \quad (I),$$

worin

$R_f$ lineares oder verzweigtes Perfluoralkyl oder Perfluoralkoxyperfluoralkyl mit 3 bis 18 C-Atomen ist,

$R_1$ lineares oder verzweigtes Alkylen, Carbamidoalkylen oder Sulfonamidoalkylen mit bis zu 6 C-Atomen darstellt, wobei das N-Atom der Amidogruppe durch Wasserstoff oder Niederalkyl substituiert ist,

m für 0,1 oder 2 steht, und

R lineares oder verzweigtes $C_2$-$C_{12}$-Alkylen ist, dadurch gekennzeichnet, dass man Merkaptan der Formel II

$$R_f-R_1-SH \quad (II),$$

worin $R_f$ und $R_1$ wie zuvor definiert sind, mit einem Alkenylglycidylether der Formel III

$$R'-OCH_2\underset{O}{CH-CH_2} \quad (III),$$

worin R' $C_2$-$C_{12}$-Alkenyl ist, umsetzt, um eine Verbindung der Formel I mit m gleich 0 zu bilden, oder dass man einen Alkohol der Formel IV

$$R_f-R_1-S(O)_m-R-OH \quad (IV),$$

worin $R_f$, $R_1$, R und m wie zuvor definiert sind, mit einem Epihalohydrin der Formel V

$$X-CH_2-\underset{O}{CH-CH_2} \quad (V),$$

worin X Halogen, bevorzugt Chlor ist, zu einem Halohydrin der Formel VI

$R_f$-$R_1$-S(O)$_m$-R-$OCH_2CH(OH)$-$CH_2X$ (VI),

worin $R_f$, $R_1$, R, m und X wie zuvor definiert sind, umsetzt, und danach die Verbindung der Formel VI zur Bildung des gewünschten Glycidylethers der Formel I dehydrohalogeniert.

10. Verwendung von Verbindungen der Formel I gemäss Anspruch 1 zur Herstellung von oberflächenaktiven, Perfluoralkylgruppen enthaltenden Substanzen.

FO 7.3/DA/cw*